# EUROPEAN PATENT APPLICATION

(11) **EP 3 050 679 A1**
(43) Date of publication of application: **03.08.2016**
(21) Application number: 16152184.4
(22) Date of filing: 21.01.2016
(51) Int. Cl.: B25J 9/00, B25J 9/12

(54) **INTEGRATED POWER GENERATION FOR HUMAN EXOSKELETONS AND METHOD OF GENERATING POWER**

(30) Priority: 28.01.2015 US 201562108757 P; 16.09.2015 US 201514856109
(71) Applicant: Steering Solutions IP Holding Corporation, Saginaw, MI 48601 (US)
(72) Inventor: Simon, Daniel C., Freeland, MI Michigan 48623 (US); Fisher, Paul M., Saginaw, MI Michigan 48603 (US); Ryne, Patrik M., Midland, MI Michigan 48642 (US); Zuraski, Jeffrey A., Saginaw, MI Michigan 48603 (US)
(74) Representative: Manitz, Finsterwald & Partner GbR

(57) **Abstract**

A human exoskeleton includes a battery. The human exoskeleton also includes an electric motor electrically coupled to the battery and powered by the battery to actuate at least one component of the human exoskeleton in an activated state of the electric motor, the electric motor back-driven during a non-activated state of the electric motor to regenerate power in the battery.

## Description

### CROSS-REFERENCES TO RELATED APPLICATIONS

This patent application claims priority to U.S. Provisional Patent Application Serial No. 62/108,757, filed January 28, 2015, which is incorporated herein by reference in its entirety.

### BACKGROUND

The embodiments described herein relate to human exoskeletons and, more particularly, to integrated power generation for human exoskeletons.

Human assist exoskeletons are a relatively new reality. These exoskeletons are intended to provide assistance in numerous contemplated manners. For example, contemplated is assistance related to walking mobility or walking assist to paraplegics, persons with disability, persons recovering from surgery or the like. They require untethered power for long periods of time to provide unhindered all-day operation to promote widespread adoption of the technology.

Many exoskeletons that are in development and/or production today have batteries integrated into their main electronic frame. This design requires a special external method to recharge the batteries. If the wearer is to continue using the device, the exoskeleton will be tethered to an electric power source until charging is completed. Such a situation lowers the usefulness of the technology and, ultimately, will greatly slow the adoption rate of the technology amongst potential users.

Exoskeleton battery replacement is tedious and cumbersome, requiring removal of the exoskeleton in many cases and/or opening of a battery pack or the like to replace the battery that powers the exoskeleton, thereby undesirably hindering continuous and easy use.

### SUMMARY OF THE INVENTION

In one embodiment of the invention, a human exoskeleton includes a battery. The human exoskeleton also includes an electric motor electrically coupled to the battery and powered by the battery to actuate at least one component of the human exoskeleton in an activated state of the electric motor, the electric motor back-driven during a non-activated state of the electric motor to regenerate power in the battery.

In another embodiment of the invention, a human exoskeleton includes a power supply electrically or chemically regenerated during a non-activated state of an actuation mechanism of the human exoskeleton.

In yet another embodiment of the invention, a method of regenerating power for a human exoskeleton is provided. The method includes activating a battery of the human exoskeleton. The method also includes supplying power from the battery to an electric motor configured to actuate at least one component of the human exoskeleton in an activated state of the electric motor. The method further includes back-driving the electric motor during a non-activated state of the electric motor and during motion of the at least one component. The method yet further includes regenerating power in the battery while back-driving the electric motor in the non-activated state.

These and other advantages and features will become more apparent from the following description taken in conjunction with the drawings.

### BRIEF DESCRIPTION OF THE DRAWINGS

The subject matter which is regarded as the invention is particularly pointed out and distinctly claimed in the claims at the conclusion of the specification. The foregoing and other features, and advantages of the invention are apparent from the following detailed description taken in conjunction with the accompanying drawings in which:
FIG. 1 is a perspective view of a human exoskeleton;
FIG. 2 illustrates the human exoskeleton worn by a user; and
FIG. 3 illustrates a method of generating power for the human exoskeleton.

### DETAILED DESCRIPTION

Referring now to the Figures, where the invention will be described with reference to specific embodiments, without limiting same, an exoskeleton 10 is configured to be strapped around the waist of a user and is also strapped to the legs of a user. The exoskeleton 10 is adapted to assist a user in leg motion. In one embodiment, the exoskeleton 10 provides an "assist" mode that is only activated by the leg motion of a user. As such, the exoskeleton 10 provides additional "muscle" to a user, allowing better mobility to a user. While the illustrated and above-described embodiment pertain to an exoskeleton device that assists with leg motion of a user, it is contemplated that exoskeleton devices that assist with motion of other body parts, such as an arm, for example, will benefit from the embodiments disclosed herein.

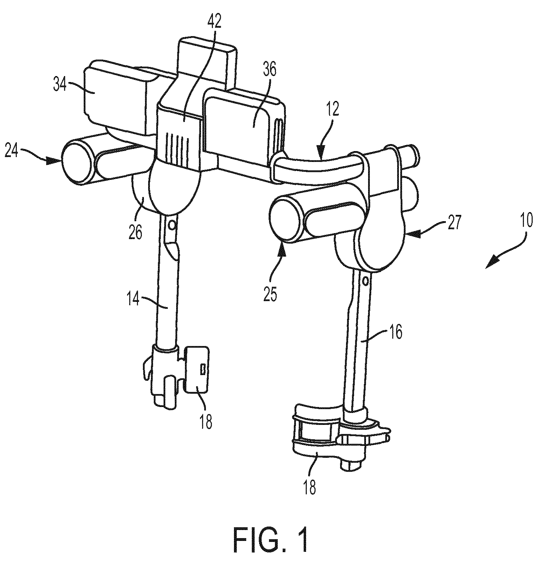

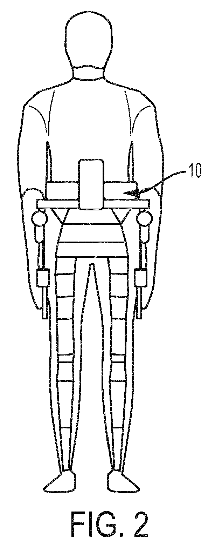

Referring to FIGS. 1 and 2, the exoskeleton 10 includes a frame 12 for fitting about the waist region of a user. In the illustrated embodiment, the frame 12 is substantially U-shaped, but alternative orientations are contemplated. The exoskeleton 10 also includes at least one leg extending from the frame 12. In the illustrated embodiment, a first leg 14 and a second leg 16 are included. Each leg 14, 16 includes a coupling device 18, such as a shackle, that connects the exoskeleton 10 to the user. The legs 14, 16 are moveable relative to the frame 12 to assist the user with mobility of the connected body parts (e.g., limbs).

The exoskeleton 10 includes an actuating device that actuates movement of the leg(s). In some embodiments, a single actuating device 24 is included and is configured to drive each of the leg(s). In the illustrated embodiment, the actuating device comprises an electric motor in the form of a first electric motor 24 and a second electric motor 25, the motors controlling each leg 14, 16, respectively. A first gearbox 26 and a second gear box 27 are operatively coupled to the actuating device (e.g., electric motor) to achieve a desired movement of the legs, 14, 16, in response to motion of an output of the actuating device, such as an output shaft of the electric motors 24, 25. The electric motors 24, 25 and the gearboxes 26, 27 are operatively coupled to the frame 12 of the exoskeleton 10.

The electric motors 24, 25 are driven by at least one power supply operatively coupled to the frame 12. In one embodiment, each electric motor 24, 25 is powered by a respective power supply, such as a first battery 34 and a second battery 36. The batteries 34, 36 may be any type of battery, such as an electric battery or an electrochemical battery, for example.

The embodiments described herein address issues with battery life that would otherwise limit a user's ability to embark on extended periods of use of the exoskeleton 10. In one embodiment, the concept of power generation through the electric motors 24, 25 and gearbox sets 26, 27. In particular, the exoskeleton 10 generates or uses electrical power interchangeably, as described in detail below.

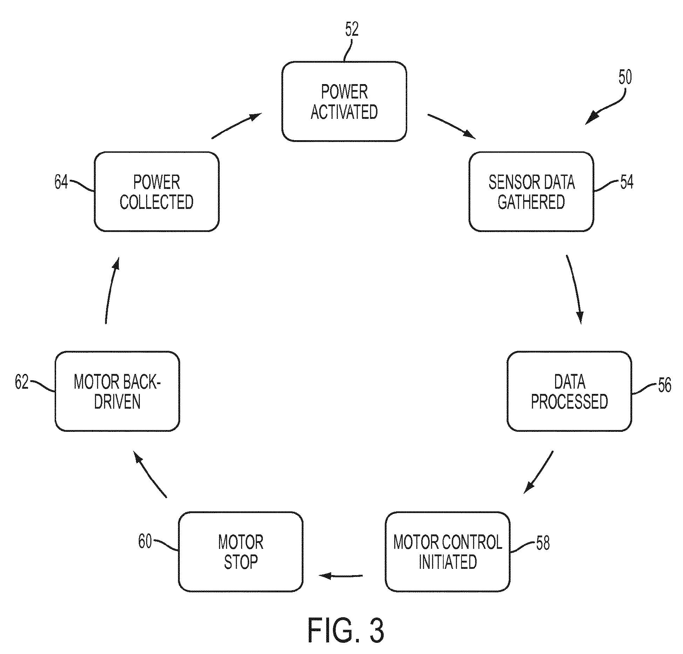

Referring now to FIG. 3, a method of controlling the exoskeleton 10 is illustrated and generally referenced with numeral 50. The exoskeleton 10 is powered on 52 and gathers data 54 using batteries 34, 36. A built-in microcontroller 42 (FIG. 1) processes 56 this data and determines how to direct 58 the motors 24, 25 and possibly the gearboxes 26, 27. If the motors 24, 25 are not activated 60 but the user is still moving, the motors 24, 25 will be back-driven 62. The motion or lack of motion of the user in the body part of interest is detected due to the operative coupling of the coupling device 18 that connects the exoskeleton 10 to the user's body part (e.g., legs), as described above. When a user uses its' own legs to move legs 14, 16, electric motors 24, 25 will be back driven. In this scenario, the electric motors 24, 25 act as a generator and the generated electrical current is directed by the microcontroller 42 back into the batteries 34, 36 as a method of regenerating power 64. This automatically recharges batteries 34 and 36 during movement of the user's legs, but without the assistance of the electric motors 24, 25.

The electrical and/or chemical power regeneration of the batteries 34, 36 in the manner described above is accomplished by eliminating an electrical hindrance that would impede the back-driving feature that enables power generation. Advantageously, power is provided to the exoskeleton 10 over an extended period of time due to the power generation capability described above.

In another aspect of the embodiments disclosed herein, replacement of the batteries 34, 36 is employed to provide an extended period of time for the user to enjoy the beneficial assistance of the exoskeleton 10. In such embodiments, the batteries 34, 36 are each coupled to the exoskeleton 10 at an exterior location such that disassembling the exoskeleton 10 is not required to access the battery mounting location. In the illustrated embodiment, the batteries 34, 36 are mounted to a rear portion of the exoskeleton 10, however, alternative regions, such as side regions, upper regions or lower regions may be utilized as battery mounting locations. Furthermore, the batteries 34, 36 are not integrated with internal locations of the frame 12 and are not disposed within a pack or other casing that hinders the user's ability to easily access the battery for replacement.

The battery may be coupled to the exterior mounting location of the exoskeleton 10 in any suitable manner that facilitates ease of removal for replacement with a replacement battery. For example, simple mechanical fasteners that are easily removed and installed with or without tools are contemplated. In this way, the connected batteries that are low or out of power are easily removed and are interchangeable with a plurality of replacement batteries while the exoskeleton remains on the user. In other words, at no time during replacement of the battery is the user required to take off the exoskeleton 10 to properly and easily conduct the replacement tasks. This allows a user to carry one or more replacement batteries in the event that the currently coupled battery runs out of its power supply.

While the invention has been described in detail in connection with only a limited number of embodiments, it should be readily understood that the invention is not limited to such disclosed embodiments. Rather, the invention can be modified to incorporate any number of variations, alterations, substitutions or equivalent arrangements not heretofore described, but which are commensurate with the spirit and scope of the invention. Additionally, while various embodiments of the invention have been described, it is to be understood that aspects of the invention may include only some of the described embodiments. Accordingly, the invention is not to be seen as limited by the foregoing description.

## Claims

1. A human exoskeleton comprising:
a battery; and
an electric motor electrically coupled to the battery and powered by the battery to actuate at least one component of the human exoskeleton in an activated state of the electric motor, the electric motor back-driven during a non-activated state of the electric motor to regenerate power in the battery.

2. The human exoskeleton of claim 1, wherein motion of the at least one component during the non-activated state back-drives the electric motor.

3. The human exoskeleton of claim 1, wherein the at least one component is a leg member operatively coupleable to a limb of a user.

4. The human exoskeleton of claim 3, wherein the limb is an arm.

5. The human exoskeleton of claim 3, wherein the limb is a leg.

6. The human exoskeleton of claim 1, wherein the battery is externally mounted to the human exoskeleton.

7. The human exoskeleton of claim 6, wherein the battery is interchangeable with a plurality of replacement batteries while the human exoskeleton remains on a user.

8. A human exoskeleton comprising a power supply electrically or chemically regenerated during a non-activated state of an actuation mechanism of the human exoskeleton.

9. The human exoskeleton of claim 8, wherein the actuation mechanism is an electric motor.

10. The human exoskeleton of claim 9, wherein the electric motor is electrically coupled to the power supply and powered by the power supply to actuate at least one component of the human exoskeleton in an activated state of the electric motor, the electric motor back-driven during the non-activated state of the electric motor to regenerate power in the battery.

11. The human exoskeleton of claim 10, wherein motion of the at least one component during the non-activated state back-drives the electric motor.

12. The human exoskeleton of claim 8, wherein the power supply is externally mounted to the human exoskeleton.

13. The human exoskeleton of claim 12, wherein the power supply is interchangeable with a plurality of replacement power supplies while the human exoskeleton remains on a user.

14. A method of regenerating power for a human exoskeleton comprising:
activating a battery of the human exoskeleton;
supplying power from the battery to an electric motor configured to actuate at least one component of the human exoskeleton in an activated state of the electric motor;
back-driving the electric motor during a non-activated state of the electric motor and during motion of the at least one component; and
regenerating power in the battery while back-driving the electric motor in the non-activated state.

15. The method of claim 14, wherein the battery is externally mounted to the human exoskeleton, the method further comprising interchanging the battery with a replacement battery while the human exoskeleton remains on a user.
